Europäisches Patentamt

(19) European Patent Office

Office européen des brevets

(11) Veröffentlichungsnummer : **0 032 675**
**B1**

(12) **EUROPÄISCHE PATENTSCHRIFT**

(45) Veröffentlichungstag der Patentschrift :
26.10.83

(21) Anmeldenummer : 81100069.4

(22) Anmeldetag : 08.01.81

(51) Int. Cl.³ : **C 12 N 15/00, C 12 P 21/02,**
**C 07 H 21/04, C 12 P 19/34,**
**C 12 N 1/20// C12R1/19**

(54) **Mikrobiologisch hergestelltes Polypeptid mit der Aminosäuresequenz des Proinsulins von Affen, DNA und Plasmide, die für diese Sequenz codieren, Mikroorganismen, die diese genetischen Informationen enthalten, und Verfahren zu deren Herstellung.**

(30) Priorität : 19.01.80 DE 3001928

(43) Veröffentlichungstag der Anmeldung :
29.07.81 Patentblatt 81/30

(45) Bekanntmachung des Hinweises auf die Patenterteilung : 26.10.83 Patentblatt 83/43

(84) Benannte Vertragsstaaten :
AT BE CH DE FR GB IT LI NL SE

(56) Entgegenhaltungen :
EP A 0 001 930
EP A 0 006 694
EP A 0 012 494
EP A 0 026 598
FR A 2 348 971
LU A 79 714

NATURE, Band 262, 29. November 1979, Seiten
525-527 G.I. BELL et al. : « Nucleotide sequence
of a cDNA clone encoding human preproinsulin »

PROC. NATL. ACAD. SCI., USA, Band 76, Nr. 8,
August 1978, Seiten 3727-3731 L. VILLA-KOMA-
ROFF et al. : « A bacterial clone synthesizing
proinsulin »

SCIENCE, Band 196, 27. June 1977, Seiten 1313-
1319 A. ULLRICH et al. : « Rat Insulin genes :
Construction of plasmids containing the coding
sequences » .

(73) Patentinhaber : HOECHST AKTIENGESELLSCHAFT
Postfach 80 03 20
D-6230 Frankfurt am Main 80 (DE)

(72) Erfinder : Wengenmayer, Friedrich, Dr.
Theodor-Körner-Strasse 12
D-6238 Hofheim am Taunus (DE)
Erfinder : Groneberg, Jürgen, Dr.
Loreleistrasse 71
D-6230 Frankfurt am Main 80 (DE)
Erfinder : Summ, Hans-Peter, Dr.
Heimchenweg 48
D-6230 Frankfurt am Main 80 (DE)
Erfinder : Winnacker, Ernst-Ludwig, Prof. Dr.
Elvirastrasse 4
D-8000 München 19 (DE)

# Mikrobiologisch hergestelltes Polypeptid mit der Aminosäuresequenz des Proinsulins von Affen, DNA und Plasmide, die für diese Sequenz codieren, Mikroorganismen, die diese genetischen Informationen enthalten, und Verfahren zu deren Herstellung

In zunehmendem Maße werden menschliche Hormone für die Therapie benötigt. Dazu gehört unter anderen das Hormon Insulin. Da es unmöglich ist, Hormone in größeren Mengen aus menschlichen Organen zu gewinnen, müssen geeignete Techniken entwickelt werden, diese Hormone außerhalb des menschlichen Organismus zu erzeugen.

Die vorliegende Erfindung zeigt einen solchen Weg auf. Sie betrifft die Isolierung von Nukleotidsequenzen, die den genetischen Code für ein spezifisches Protein enthalten, die Synthese von DNA mit diesen spezifischen Nukleotidsequenzen und den Transfer dieser DNA in einen Mikroorganismus als Wirt, in dem die Replikation und Expression dieser DNA erfolgt.

Es ist bekannt, Polypeptide und Proteine durch Kultivieren von solchen Bakterien herzustellen, die Plasmide mit Genen tragen, die für die gewünschten Polypeptide oder Proteine codieren. Ferner ist bekannt, daß Plasmide konstruiert werden können, die den sie tragenden Wirt dazu veranlassen, Genprodukte herzustellen, die von Natur aus nicht für diesen charakteristisch sind. Es ist auch bekannt, daß durch DNA-Rekombinationstechniken die genetische Information von Ratten-Präproinsulin in Bakterien kloniert wurde. Es konnte gezeigt werden, daß diese Bakterien Protein exprimieren, das Antigen-Determinanten von Insulin besitzt. Darüber hinaus ist bekannt, daß die genetische Information von A- und B-Kette des Menscheninsulins getrennt in Bakterien kloniert wurde. Dieses wurde dadurch erreicht, daß die entsprechenden DNAs für A- und B-Kette de novo synthetisiert wurden. Die durch die Bakterien produzierten A- und B-Ketten konnten dann in vitro zum Insulinmolekül verknüpft werden.

Keiner dieser Wege hat zu einem in der menschlichen Therapie verwendbaren Pharmakon geführt. Im Falle des Rattenproinsulins ist das Endprodukt, das Ratteninsulin, ein Eiweiß, das zu allergischen Reaktionen führt. Im Falle der Human-A- und B-Kette stehen die Eigenschaften der B-Kette wegen ihrer Polymerisationsneigung und die sehr schlechte Ausbeute bei der Kombination von A- und B-Kette dem Endziel, Humaninsulin, entgegen.

Mit der vorliegenden Erfindung wird daher ein anderer Weg eingeschlagen. Es ist bekannt, daß Affen- und Menscheninsulin eine identische Struktur besitzen. Wenn es gelingt, Affenproinsulin herzustellen, kann man daraus durch Spaltung Menscheninsulin gewinnen.

Die vorliegende Erfindung betrifft nun im besonderen die Darstellung von Genen für Affenproinsulin, den Transfer dieser Gene in einen mikrobiellen Wirt, Replikation von Wirt und Gen und Expression des Gens durch den Wirt. Dabei entstehen Proteine, die die Aminosäuresequenzen des Affenproinsulins enthalten.

Zur Erreichung des erfindungsgemäßen Zieles kann man in folgender Weise verfahren, wobei der aufgezeichnete Weg beispielhaft für mehrere begangene steht.

Man gewinnt aus Affenpankreata, vorzugsweise vom Rhesusaffen oder Cynomolgen, durch proteolytische Verdauung, die wegen der möglichen weitergehenden Zerstörung sehr sorgfältig kontrolliert werden muß, und anschließende Zentrifugation Einzelzellen. Aus diesen wird durch Dichtezentrifugation über einen Gradienten eine an β-Zellen reiche Schicht gewonnen. Diese Zellschicht wird denaturiert und die RNA als Niederschlag nach einer Cäsiumchloridzentrifugation gewonnen. Nach weiterem Umfällen wird aus dieser RNA die mRNA über eine Oligo-dT-Cellulosesäule abgetrennt. Die gewonnene mRNA wird mit Hilfe des Enzyms Reverse Transcriptase zu einem RNA-DNA-Doppelstrang komplettiert. Nach Verdauung des RNA-Stranges wird der DNA-Strang (die komplementäre DNA = cDNA) mit Reverser Transkriptase oder dem Enzym Polymerase I zum DNA-DNA-Doppelstrang (dsRNA) komplettiert.

Diese doppelsträngige DNA muß nun in ein Plasmid eingebaut werden. Dazu ist die Verlängerung des 3'-Endes der DNA z. B. mit dCMP-Resten notwendig. Das Plasmid (z. B. pBR 322) wird mit der Restriktionsnuklease PstI aufgeschnitten und z. B. mit dGMP-Resten verlängert. Bei Zusammengabe der so verlängerten DNA mit dem entsprechend verlängerten Plasmid erfolgt Basenpaarung zwischen DNA und Plasmid. Dieses zirkularisierte Molekül wird dann in Mikroorganismen (vor allem Bakterien, vorzugsweise E. coli, wie E. coli K 12 oder χ 1776) transformiert; die noch nicht geschlossenen Bindungen werden durch die Enzyme des Mikroorganismus kovalent geknüpft. Die transformierten Mikroorganismen, werden auf Agarplatten ausplattiert und auf Antibiotikaresistenzen, die durch das gewählte Plasmid und die eingesetzte Restriktionsnuklease gegeben sind, selektioniert.

Mit Hilfe immunologischer Teste werden die Klone, die insulinhaltige Proteine exprimieren, herausgesucht. Diese Klone werden gezüchtet, die Mikroorganismenmasse abzentrifugiert, extrahiert und auf Insulingehalt getestet. Hierbei konnten in einer Reihe von Klonen Insulinwerte von über einer Insulineinheit (= 1 IE) pro Liter Kulturlösung gemessen werden. Diese Kulturlösung dient als Ausgangsmaterial für die Gewinnung von Affenproinsulin und nachfolgend Humaninsulin.

Die erfindungsgemäßen Verfahren können in folgender Weise durchgeführt weren :

1. Verfahren zur Isolierung von RNA

a) Die Isolierung von insulinproduzierenden Pankreaszellen

Von Affen, vorzugsweise Rhesus-Affen oder Cynomolgen, werden die Pankreata unter sterilen Bedingungen gewonnen und nach Beseitigung der Häute und größerer Blutgefäße zunächst mechanisch mittels Scheren zerkleinert. Dabei ist darauf zu achten, daß das Gewebe während der Arbeiten kühl gehalten wird, um einen Selbstaufschluß infolge des darin enthaltenen Pankreatin-Enzymgemisches zu vermeiden. Das zerkleinerte Gewebe wird mehrmals mit gekühltem Säugetierserum, vorzugsweise Kälberserum, gewaschen, danach in Kölbchen gegeben und mit einer sterilen 0,125 %igen erwärmten Collagenaselösung in PBS versetzt. Die Collagenaselösung ist stets frisch vor dem Aufschluß anzusetzen. Entscheidend für die Ausbeute an Zellen aus Pankreas ist die gleichzeitige Zugabe von 20 und mehr % Serum, vorzugsweise Kälberserum, zur Collagenase. Dieses Gemisch wird 10 Minuten gerührt und der gesamte Überstand zunächst verworfen. Danach wird erneut ein Collagenase-Serumgemisch zugesetzt und etwa 6 bis 10 Minuten gerührt. Dieser Vorgang wird etwa 10 mal wiederholt und der jeweils gewonnene Aufschluß im Kühlbehalter gesammelt. Das gesammelte Material mit den enzymatisch gewonnenen Zellen wird bei etwa 1 000 upm 10 Minuten in einer Kühlzentrifuge zentrifugiert, der Überstand verworfen und der Zellbodensatz in frischem Dulbecco-Medium mit 1 g/l Glukose und 20 % Kälberserum, resuspendiert. Diese Zentrifugation wird wiederholt und der danach gewonnene Bodensatz in 10 ml Dulbecco-Medium mit 15 % Serumzusatz resuspendiert.

Dieses Zellmaterial, das aus einem Gemisch von β-Zellen, Fibroblasten und epitheloiden Zellen besteht, wird anschließend über einen Dichtegradienten, beispielsweise aus Ficole oder Percoll, aufgetrennt. Am besten bewährt hat sich hierzu ein Ficoll-Dichtegradient. Dazu wurde Ficoll 400/Pharmacia in Dulbecco-Medium ohne Serum gelöst und im Refraktometer auf eine Viskosität von 1,38 oder mit der Spindel auf eine Dichte von 1,16 eingestellt, durch ein Membranfilter mit einer Porenweite von 0,45 μ sterilfiltriert und bei + 4 °C bis zur Verwendung aufbewahrt. Von dieser Ficollausgangssuspension werden Dichten mit einer Viskosität von 1,373, 1,370 und 1,3672 durch Zugabe von Dulbecco-Medium eingestellt und im Zentrifugenröhrchen übereinandergeschichtet. Obenauf werden die resuspendierten Pankreaszellen gegeben und 10 Minuten bei + 4 °C und 3 200 upm zentrifugiert. Nach der Zentrifugation werden die einzelnen Banden vorsichtig gewonnen und von restlichem Ficoll mit Dulbecco-Medium freigewaschen. Die Hauptmasse der insulinproduzierenden β-Zellen wird auf den obersten Schichten mit der geringsten Dichte und einer Viskosität von 1,3672 angereichert.

b) Gewinnung von RNA

Die erhaltenen Zellen werden in « denaturierendem Medium » (4 M Guanidiniumthiocyanat, 1 M Mercaptoäthanol, 0,15 M Natriumacetat — pH 5,5) aufgenommen und 1 Minute bei 0 °C mit dem Ultra-Turrax homogenisiert. Die Lösung wird 5 Minuten auf 80 °C erwärmt und sofort in ein Eisbad gegeben.

Diese wird dann auf eine Lösung (« Kissen ») aus 5 ml 5,7 M CsCl, 10 mM Tris-hydroxiaminomethan (Tris), 10 mM Äthylendiamintetraessigsäure (EDTA) vom pH 7,6 geschichtet und in einem Beckman-SW 27 Rotor 36 Stunden bei 20 °C und 22 000 UpM zentrifugiert. Es wurde gefunden, daß, im Gegensatz zu Angaben in der Literatur, ein Zusatz von CsCl zu dem Lysat vermieden werden muß, um eine möglichst vollständige Abtrennung der mRNA im folgenden Reaktionsschritt zu erreichen. Nach beendeter Zentrifugation werden die Polyallomer-Röhrchen in flüssigem Stickstoff eingefroren und der Boden des Röhrchens, auf dem sich der RNA-Niederschlag befindet, abgeschnitten. Der Niederschlag wird in einer Lösung aus 10 mM Tris, 10 mM EDTA und 1 % « Sarkosyl » (N-Lauryl-Sarkosin-Na-Salz) vom pH 7,6 aufgenommen und die Suspension bei 20 000 UpM 20 Minuten abzentrifugiert. Der erhaltene Niederschlag wird nochmals in dem gleichen Puffer aufgenommen, 5 Minuten auf 65 °C erwärmt und erneut zentrifugiert. Die vereinigten Überstände werden 0,3 M an Natriumacetat gemacht, mit dem 2,5-fachen Volumen an Äthanol versetzt und bei − 20 °C aufbewahrt.

2. Gewinnung von mRNA

Die RNA wird durch Abzentrifugation aus der äthanolischen Lösung (20 000 UpM, 30 Minuten, − 5 °C) abgetrennt und in 0,5 M NaCl, 10 mM Tris pH 7,5 gelöst. Diese Lösung wird auf eine Oligo-dT-Cellulose-Säule (Hersteller : Collaborative Research, Type 3), die mit dem gleichen Puffer äquilibriert ist, aufgetragen und mit 1 mM Tris pH 7,6 oder destilliertem Wasser eluiert. Die Elution der Poly-A-haltigen RNA (mRNA) kann durch Extinktionsmessung bei 260 nm verfolgt werden. Die so erhaltene mRNA wird mit Natriumacetat bis zu einer Endkonzentration von 0,3 M versetzt, das 2,5-fache Volumen an Äthanol zugegeben und die Lösung bei − 20 °C aufbewahrt.

3. Gewinnung der cDNA

a) Gewinnung von Einzelstrang-cDNA

Die Übertragung der mRNA in die entsprechende DNA (cDNA) erfolgt mit Hilfe des Enzyms Reverser Transcriptase. Der Inkubationsansatz enthält :

50 mM Tris, pH 8,3, 10 mM $MgCl_2$, 30 mM 2-Mercaptoäthanol, 0,5 mM an allen 4 üblicherweise vorkommenden Desoxyribonukleosidtriphosphaten (entsprechende Triphosphate von Adenin, Guanin, Cytosin und Thymidin), 100 μg/ml Oligo-$dT_{12-18}$ (Hersteller : Boehringer Mannheim) etwa 100 μg/ml polyadenylierte RNA und 800 Einheiten/ml Reverser Transcriptase (Hersteller : Life Science Inc., St. Petersburg, USA). Zur Verfolgung der Reaktion kann ein in α-Stellung mit $^{32}$P-markiertes Desoxyribonukleosidtriphosphat (spez. Aktiv. : 50 Ci/mMol) zum Ansatz gegeben werden. Das Gemisch wird 60 Minuten bei 42 °C inkubiert, worauf durch Zusatz von 20 mM EDTA die Reaktion abgestoppt mit Wasser gesättigtem Phenol extrahiert, das Phenol durch Ausschütteln mit Äther entfernt und Reste Äther mit Stickstoff abgedampft. Nicht eingebaute Desoxyribonukleosidtriphosphate werden durch Säulenchromatographie an Sephadex G50 in 10 mM Tris, pH 9,0, 100 mM NaCl, 1 mM EDTA abgetrennt. Die eluierte cDNA wird durch Zusatz von 0,3 M natriumacetat und dem 2,5-fachen Volumen Äthanol ausgefällt.

Nach Zentrifugation wird der Niederschlag in 0,1 M NaOH aufgenommen und 20 Minuten bei 70 °C inkubiert oder in 0,3 M NaOH über Nacht bei Raumtemperatur inkubiert. Das Gemisch wird mit 1 M HCl und 1 M Tris auf pH 7,6 gebracht.

b) Bildung von Doppelstrang-cDNA (dsDNA)

Zur Synthese des zweiten DNA-Stranges kann wiederum das Enzym Reverse Transkriptase oder das Enzym Polymerase I verwendet werden.

Bildung der dsDNA mit Reverser Transcriptase :

Der Inkubationsansatz (pH 8,3) enthält 50 mM Tris, 10 mM $MgCl_2$, 30 mM 2-Mercaptoäthanol, 0,5 mM der oben genannten 4 Desoxyribonukleosidtriphosphate, 50 μg/ml cDNA und 800 Einheiten/ml Reverser Transcriptase. Die Reaktion wird 120 Minuten bei 42 °C durchgeführt und durch Zusatz von EDTA bis zur Endkonzentration von 20 mM abgestoppt. Anschließend erfolgt die Phenol-Extraktion und Gelpermeationschromatographie an Sephadex G50 wie vorher beschrieben.

Bildung der dsDNA mit Polymerase I :

Der Inkubationsansatz (pH 6,9) enthält 200 mM Hepes, 0,5 mM der obengenannten 4 Desoxyribonukleosidtriphosphate, 10 mM $MgCl_2$, 30 mM 2-Mercaptoäthanol und 70 mM KCl. Durch Zusatz von $^{32}$P-markierten Desoxyribonukleotiden (an den α-Positionen) zum Inkubationsansatz kann die Reaktion verfolgt werden. Die Reaktion wird mit 800 Einheiten/ml Polymerase I gestartet und 2 Stunden bei 15 °C durchgeführt. Durch Zusatz von 0,1 % Natriumdodecylsulfat und 100 μg/ml RNA wird die Reaktion abgestoppt. Die Lösung wird wie beschrieben extrahiert. Die nach der Extraktion erhaltene Lösung wird auf eine Sephadex G50 Säule aufgetragen und die Chromatographie mit 10 mM Tris pH 9,0, 100 mM NaCl und 1 mM EDTA durchgeführt. Die eluierte DNA kann durch die Radioaktivität bestimmt werden und wird durch Zusatz von 0,3 M Natriumacetat und 2,5-fachem Volumen Äthanol gefällt. Der Äthanol-Niederschlag wird in einer Lösung (pH 4,5) aus 300 mM NaCl, 30 mM Natriumacetat und

3 mM $ZnCl_2$ aufgenommen und mit 1 500 Einheiten/ml S1 Nuklease (Boehringer Monnheim) versetzt. Die Reaktion wird nach einer Stunde bei 37 °C durch Zusatz von EDTA bis zu einer Endkonzentration von 20 mM beendet. Anschließend erfolgt Extraktion mit Phenol und Fällung mit Äthanol wie beschrieben.

c) Gewinnung von cDNA in einem « Eintopfverfahren »

Alternativ zu den beschriebenen Reaktionen kann die dsDNA aus der mRNA auch in einem « Eintopfverfahren » gewonnen werden. Dabei wird die Reaktion der Reversen Transcriptase wie beschrieben durchgeführt, zum Inkubationsansatz wird allerdings noch 140 mM KCl hinzugefügt. Nach beendeter Reaktion wird die Probe 4 Minuten auf 100 °C erhitzt und der gebildete Niederschlag abzentrifugiert. Zu dem Überstand wird das gleiche Volumen an 0,4 M Hepes Puffer (pH 6,9) gegeben, wobei die oben genannten 4 Desoxyribonukleotide in dem Puffer 0,5 mM vorhanden sind. Durch Zugabe von 800 Einheiten/ml Polymerase I wird die Reaktion wie beschrieben bei 15 °C durchgeführt und durch Zusatz von Natriumdodecylsulfat und RNA abgestoppt. Anschließend wird wie unter 3.b) « Bildung der dsDNA mit Polymerase I" weiter verfahren.

4. Verlängerung der 3'-Enden von cDNA mit dCTP (Desoxicytosintriphosphat)

Zum Einbau in das Plasmid ist die Verlängerung des 3'-Endes der DNA mit einem der oben genannten 4 Desoxynukleotide notwendig. Ferner werden die 3'-Enden des geschnittenen Plasmids mit dem komplementären Desoxynukleotid verlängert. Bei Zusammengabe der DNA mit dem Plasmid erfolgt dann eine Basenpaarung zwischen DNA und Plasmid. Dieses wieder zirkularisierte Molekül kann zur Transformation von Bakterien eingesetzt werden ; die noch nicht geschlossenen Bindungen werden durch ein Enzymsystem im Bakterium kovalent verknüpft.

Beispielsweise können die 3'-Enden der cDNA mit dCMP-Resten (Desoxicytosinmonophosphatreste) und das Plasmid mit dGMP-Resten (Desoxiguanidinmonophosphat-Reste) verlängert werden. Bei Verwendung dieser Desoxynukleotide in der beschriebenen Weise und bei Öffnung des Plasmids durch das Restriktionsenzym Pst I wird nach Einbau der fremden DNA eine Pst I-Spaltstelle an jeder Insertionsstelle neu gebildet, so daß nach Vermehrung des Plasmids die insertierte DNA zur weiteren Untersuchung leicht wieder mit dem Restriktionsenzym Pst I herausgeschnitten werden kann.

Der Niederschlag aus der Alkohol-Fällung nach Abbau mit S1 Nuklease wird in einer wäßrigen Lösung (pH 6,7) aus 30 mM Tris, 1 mM $CoCl_2$, 140 mM Kalium-Kacodylat, 150 μM dCTP, 100 μg autoklavierten Gelatinehydrolisats und 0,1 M Dithioerythrit gelöst. Zur Verfolgung der Reaktion

sollte $^{32}$P-markiertes dCTP verwendet werden. Die Reaktion wird durch Zusatz von Terminaler Desoxynukleotidyl-Transferase gestartet und für 10 Minuten bei 37 °C durchgeführt. Nach Ablauf der Reaktionszeit wird die Probe in Eis gestellt und durch Radioaktivitätsmessung im mit Trichloressigsäure fällbaren Niederschlag die Anzahl der eingebauten dCMP-Reste bestimmt. Bei der Reaktion sollten etwa 10 % der ursprünglich vorhandenen Nukleotide addiert worden sein ; wenn dies nicht der Fall ist, kann durch Zusatz von weiterem Enzym die Reaktion fortgesetzt werden. Falls eine zu große Anzahl von dCMP-Resten addiert wurde, kann die entstandene Kette mit Hilfe des Enzyms S1 Nuklease verkürzt werden.

### 5. Verfahren zur Integration von DNA in ein Plasmid

Ein geeignetes zirkuläres Plasmid, beispielsweise pBR 322, wird mit einer restriktionsendonuklease geschnitten, welche nur eine Sequenz auf dem Plasmid erkennt. Günstig ist es, wenn diese Spaltstelle hinter einem starken oder induzierbaren Promotor liegt und/oder so lokalisiert ist, daß eine Antibiotikaresistenz beeinflußt wird.

Das solchermaßen linearisierte Plasmid wird dann an den 3'-Enden um eines der vier Desoxynukleotide verlängert.

Dies wird z. B. folgendermaßen durchgeführt : 30 µg Plasmid werden mit 50 Einheiten Pst I Restriktionsendonuklease in Gegenwart von 50 mM NaCl, 6 mM Tris, 6 mM $MgCl_2$, 6 mM 2-Mercaptoäthanol und 0,1 mg/ml Gelatine für 40 Minuten bei 37 °C und einem pH-Wert von 7,5 inkubiert. Anschließend wird mit Phenol und Äther wie beschrieben extrahiert und das geschnittene Plasmid mit Äthanol in Gegenwart von 0,3 M Natriumacetat gefällt. Die Verlängerung der 3'-Enden des Plasmids mit dGTP (Desoxiguanidintriphosphat) erfolgt analog zu der oben beschriebenen Verlängerung der cDNA mit dCTP.

Die verlängerte Plasmid-DNA wird dann in einer Lösung (pH 8,0) aus 0,1 M NaCl, 10 mM Tris und 1 mM EDTA mit der verlängerten ds cDNA vermischt, für 2 Minuten auf 56 °C erwärmt, 2 Stunden bei 42 °C inkubiert und dann langsam auf Raumtemperatur abgekühlt. Die solchermaßen erhaltene hybride DNA wird dann zur Transformation von E. coli verwendet.

### 6. Klonierung des Hybridplasmids in E. coli

Die Bakterien, beispielsweise E. coli χ 1776, wurden bei 37 °C in 50 ml eines üblichen Nährmediums bis zu einer optischen Dichte von $A_{600} = 0,5-0,6$ wachsen gelassen, sedimentiert, einmal mit 10 mM Tris pH 7,5 gewaschen und dann in 40 ml einer Lösung aus 75 mM $CaCl_2$, 5 mM $MgCl_2$ und 5 mM Tris vom pH 8,0 resuspendiert und 20 Minuten in Eis inkubiert. Dann wurden die Zellen sedimentiert und in 2 ml 75 mM $CaCl_2$, 5 mM $MgCl_2$, 5 mM Tris pH 8,0 resuspendiert.

0,2 ml dieser Bakteriensuspension wurden dann mit 0,1 ml Hybrid-DNA-Lösung vermischt und 45 Minuten auf Eis inkubiert. Dann wurde für 90 Sekunden auf 42 °C erwärmt und anschließend mit 0,2 ml Nährmedium vermischt.

50 µl-75 µl dieser Suspension wurden dann auf Tetracyclin und Ampicillin enthaltende Agarplatten ausplattiert und auf Antibiotikaresistenz selektioniert.

### 7. Isolierung von Stämmen, die insulinenthaltende Proteine produzieren

Mit Hilfe eines von Broome und Gilbert (Broome, S. und Gilbert, E. PNAS *75*, 2746, 1978) entwickelten Testsystems wurden die Klone auf die Expression insulinenthaltender Proteine untersucht. Klone, die insulinenthaltende Proteine exprimieren, werden durch die Bindung von radioaktivem Antikörper an diese Proteine mit nachfolgender Autoradiographie dadurch erkenntlich, daß der Röntgenfilm an dieser Stelle geschwärzt wird.

Dazu wurden bis zu 50 Klone pro Nitrocellulosefilter für 2 Tage bei 37 °C wachsen gelassen. Dann wurden die Filter auf einen Agarblock gelegt, welcher 1 mg/ml Lysozym enthielt ; auf die Bakterienkolonie wurde eine mit Insulinantikörper beschichtete PVC-Folie aufgebracht, und dann wurde für 2-3 Stunden bei 4 °C inkubiert. Die PVC-Folie wurde anschließend für 15 Stunden bei 4 °C in einer Lösung von [132]Jodmarkiertem Insulinantikörper inkubiert. Die spezifische Aktivität der Lösung war ca. $1 \times 10^6$ cpm/ml. Nach dem Waschen und Trocknen der Folien wurden diese autoradiographiert.

Die auf der Platte immunologisch auf Insulin reagierenden Klone werden in einem üblichen Nährmedium gezüchtet, die Bakterien abzentrifugiert, extrahiert und nun im Radioimmunoassay auf Insulingehalt ausgetestet. Eine Anzahl von Klonen zeigen dabei Insulinwerte von mehr als 1 IE pro Liter Kulturlösung.

**Ansprüche** (für die Vertragsstaaten : BE, CH, DE, FR, GB, IT, LI, NL, SE)

1. Mikrobiologisch hergestelltes Polypeptid, das die Aminosäuresequenz des Proinsulins von Affen enthält.

2. Mikrobiologisch hergestelltes Polypeptid nach Anspruch 1, das die Aminosäuresequenz des C-Peptids des Proinsulins von Affen enthält.

3. cDNA, die für Aminosäuresequenzen des Proinsulins gemäß Ansprüchen 1 bis 2 codiert, hergestellt mittels mRNA von Affen.

4. Plasmid, das für Aminosäuresequenzen gemäß Ansprüchen 1 bis 2 codiert, hergestellt aus der cDNA gemäß Anspruch 3 und einem Plasmid, vorzugsweise dem Plasmid pBR 322.

5. Mikroorganismus, insbesondere E. coli, mit der genetischen Information für die Biosynthese des Polypeptids von Ansprüchen 1 bis 2.

6. Verfahren zur Herstellung des Polypeptids gemäß den Ansprüchen 1 bis 2, dadurch gekennzeichnet, daß man die genetische Information für die Biosynthese des Polypeptids mittels mRNA von Affen gewinnt, das erhaltene Gen in an sich bekannter Weise in Mikroorganismen einbringt und diejenigen Mikroorganismen in an sich bekannter Weise herausselektioniert und kultiviert, die dieses Polypeptid produzieren.

7. Verfahren zur Herstellung von cDNA nach Anspruch 3, dadurch gekennzeichnet, daß man aus der Pankreas von Affen RNA gewinnt, daraus die mRNA isoliert und mittels dieser in an sich bekannter Weise cDNA herstellt.

8. Verfahren nach Anspruch 7, dadurch gekennzeichnet, daß man bei der Gewinnung von Inselzellen aus dem Pankreas von Affen Säugetierserum, vorzugsweise Kälberserum, in einer Menge von vorzugsweise 20 oder mehr Prozent des Gesamtvolumens zusetzt und aus den so gewonnenen Inselzellen RNA gewinnt.

9. Verfahren zur Herstellung eines Mikroorganismus gemäß Anspruch 5, dadurch gekennzeichnet, daß man einen Mikroorganismus, vorzugsweise E. coli, mit einem Plasmid gemäß Anspruch 4 transformiert.

## Ansprüche (für den Vertragsstaat AT)

1. Verfahren zur Herstellung eines Polypeptids, dadurch gekennzeichnet, daß man einen Mikroorganismus, der die genetischen Informationen für die Biosynthese des Polypeptids mit der Aminosäuresequenz des Proinsulins von Affen enthält, kultiviert und das von dem Mikroorganismus gebildete Polypeptid isoliert.

2. Verfahren nach Anspruch 1, dadurch gekennzeichnet, daß man einen Mikroorganismus, der die genetischen Informationen für die Biosynthese des Polypeptids mit der Aminosäuresequenz des C-Peptids des Proinsulins von Affen enthält, kultiviert und das zu erzeugte Polypeptid isoliert.

3. Verfahren zur Herstellung des Polypeptids gemäß Ansprüchen 1-2, dadurch gekennzeichnet, daß man die genetischen Informationen für die Biosynthese des Polypeptids mittels mRNA von Affen gewinnt, das erhaltene Gen in an sich bekannter Weise in Mikroorganismen einbringt und diejenigen Mikroorganismen herausselektioniert und kultiviert, die das Polypeptid produzieren.

4. Verfahren zur Herstellung von cDNA, die für Aminosäuresequenzen des Proinsulins von Affen codiert, dadurch gekennzeichnet, daß man aus der Pankreas von Affen RNA gewinnt, daraus die mRNA isoliert und mittels dieser in an sich bekannter Weise cDNA herstellt.

5. Verfahren nach Anspruch 4, dadurch gekennzeichnet, daß man bei der Gewinnung von Inselzellen aus dem Pankreas von Affen Säugetierserum, vorzugsweise Kälberserum, in einer Menge von 20 oder mehr Prozent des Gesamtvolumens zusetzt und aus den so gewonnenen Inselzellen RNA gewinnt.

6. Verfahren zur Herstellung eines Plasmids, das für die Aminosäuresequenzen des Proinsulins von Affen codiert, dadurch gekennzeichnet, daß man die nach Anspruch 4 hergestellte cDNA mit einem Plasmid, vorzugsweise dem Plasmid pBR 322, kombiniert.

7. Verfahren zur Herstellung des gemäß Ansprüchen 1 bis 2 eingesetzten Mikroorganismus, dadurch gekennzeichnet, daß man einen Mikroorganismus, vorzugsweise E. coli, mit einem Plasmid, das gemäß Anspruch 6 hergestellt wurde, transformiert.

## Claims (for the Contracting States : BE, CH, DE, FR, GB, IT, LI, NL, SE)

1. A microbiologically prepared polypeptide which contains the aminoacid sequence of the proinsulin of monkeys or apes.

2. A microbiologically prepared polypeptide according to claim 1 which contains the aminoacid sequence of the C-peptide of the proinsulin of monkeys or apes.

3. A cDNA coding for aminoacid sequences of the proinsulin according to claims 1 to 2, prepared by means of mRNA of monkeys or apes.

4. A plasmid coding for aminoacid sequences according to claims 1 to 2, prepared from the cDNA according to claim 3 and from a plasmid, preferably the plasmid pBR 322.

5. A microorganism, in particular E. coli, with the genetic information for the biosynthesis of the polypeptide of claims 1 to 2.

6. A process for the preparation of the polypeptide according to claims 1 to 2, characterized by obtaining the genetic information for the biosynthesis of the polypeptide by means of mRNA of monkeys or apes, incorporating the obtained gene into microorganisms in a manner which is in itself known, and selecting and culturing, in a manner which is in itself known, those microorganisms which produce this polypeptide.

7. A process for the preparation of cDNA according to claim 3, characterized by obtaining RNA from the pancreas of monkeys or apes, isolating the mRNA there-from and preparing by means of the latter cDNA in a manner which is in itself known.

8. A process according to claim 7, characterized in that in the preparation of islet cells from the pancreas of monkeys or apes, mammal serum, preferably calf serum, is added in an amount of preferably 20 or more per cent of the total volume, and RNA is obtained from the islet cells prepared in this way.

9. A process for the preparation of a microorganism according to claim 5, characterized by transforming a microorganism, preferably E. coli, with a plasmid according to claim 4.

## Claims (for the Contracting State AT)

1. A process for preparing a polypeptide, characterized by culturing a microorganism containing the genetic information for the biosynthesis of the polypeptide with the aminoacid sequence of proinsulin of monkeys or apes and isolating the polypeptide formed by the microorganism.

2. A process according to claim 1, characterized by culturing a microorganism containing the genetic information for the biosynthesis of the polypeptide with the aminoacid sequence of the C-peptide of proinsulin of monkeys or apes and isolating the polypeptide formed.

3. A process for preparing the polypeptide according to claims 1 to 2, characterized by obtaining the genetic information for the biosynthesis of the polypeptide by means of mRNA of monkeys or apes, introducing the obtained gene into microorganisms in a manner which is in itself known and selecting and culturing those microorganisms which produce the polypeptide.

4. A process for preparing cDNA coding for the aminoacid sequences of proinsulin of monkeys or apes, characterized by obtaining RNA from the pancreas of monkeys or apes, isolating therefrom mRNA and producing by means of the latter cDNA in a manner which is in itself known.

5. A process according to claim 4, characterized in that in the preparation of islet cells from the pancreas of monkeys or apes, mammal serum, preferably calf serum, is added in an amount of preferably 20 or more per cent of the total volume, and RNA is obtained from the islet cells prepared in this way.

6. A process for preparing a plasmid coding for the aminoacid sequences of proinsulin of monkeys or apes, characterized by combining cDNA prepared according to claim 4 with a plasmid, preferably the plasmid pBR 322.

7. A process for preparing the microorganism employed according to claims 1 to 2, characterized by transforming a microorganism, preferably E. coli, with a plasmid prepared according to claim 6.

**Revendications** (pour les Etats Contractants : BE, CH, DE, FR, GB, IT, LI, NL, SE)

1. Polypeptide préparé microbiologiquement, qui contient la séquence en amino-acides de la proinsuline de singes.

2. Polypeptide préparé microbiologiquement selon la revendication 1, qui contient la séquence en amino-acides du peptide C de la proinsuline de singes.

3. cADN codant pour les séquences en amino-acides de la proinsuline selon l'une des revendications 1 et 2, préparé au moyen de mARN de singes.

4. Plasmide codant pour les séquences en amino-acides selon l'une des revendications 1 et 2, préparé à partir du cADN selon la revendication 3 et d'un plasmide, de préférence le plasmide pBR 322.

5. Microorganisme, en particulier E. coli, avec l'information génétique pour la biosynthèse du polypeptide selon l'une des revendications 1 et 2.

6. Procédé pour la préparation du polypeptide selon l'une des revendications 1 et 2, caractérisé en ce qu'on obtient l'information génétique pour la biosynthèse du polypeptide au moyen de mARN de singes, on introduit le gène dans des microorganismes d'une manière connue en soi et on sélectionne et on cultive d'une manière connue en soi les microorganismes qui produisent ce polypeptide.

7. Procédé pour la préparation de cADN selon la revendication 3, caractérisé en ce qu'on produit de l'ARN à partir du pancréas de singes, on en isole le mARN et au moyen de ce dernier on prépare du cADN d'une manière connue en soi.

8. Procédé selon la revendication 7, caractérisé en ce que pour la préparation de cellules isolées à partir du pancréas de singes, on ajoute un sérum de mammifères, de préférence du sérum de veau, en une quantité qui est avantageusement de 20 % ou plus du volume total et on obtient l'ARN à partir de ces cellules isolées ainsi obtenues.

9. Procédé pour la préparation d'un microorganisme selon la revendication 5, caractérisé en ce qu'on transforme un microorganisme, de préférence E. coli, avec un plasmide selon la revendication 4.

**Revendications** (pour l'Etat Contractant AT)

1. Procédé pour la préparation d'un polypeptide, caractérisé en ce qu'on cultive un microorganisme qui contient les informations génétiques pour la biosynthèse du polypeptide avec la séquence en amino-acides de la proinsuline de singes, et à partir du microorganisme on isole le polypeptide formé.

2. Procédé selon la revendication 1, caractérisé en ce qu'on cultive un microorganisme qui contient les informations génétiques pour la biosynthèse du polypeptide avec la séquence en amino-acides du peptide C de la proinsuline de singes, et on isole le polypeptide produit.

3. Procédé pour la préparation du polypeptide selon l'une des revendications 1 et 2, caractérisé en ce qu'on obtient les informations génétiques pour la biosynthèse du polypeptide au moyen de mARN de singes, on introduit le gène obtenu, d'une manière connue en soi, dans des microorganismes et on sélectionne et on cultive les microorganismes qui produisent le polypeptide.

4. Procédé pour la préparation de cADN codant pour les séquences en amino-acides de la proinsuline de singes, caractérisé en ce qu'on produit de l'ARN à partir du pancréas de singes, on en isole le mARN et au moyen de ce dernier on prépare du cADN d'une manière connue en soi.

5. Procédé selon la revendication 4, caractérisé en ce que pour la préparation de cellules isolées à partir du pancréas de singes, on ajoute un sérum de mammifères, de préférence du sérum de veau, en une quantité de 20 % ou plus du volume total, et on produit l'ARN à partir des cellules isolées ainsi obtenues.

6. Procédé pour la préparation d'un plasmide codant pour la séquence en amino-acides de la proinsuline de singes, caractérisé en ce qu'on combine le cADN obtenu selon la revendication 4, avec un plasmide, de préférence le plasmide pBR 322.

7. Procédé pour la préparation du microorganisme utilisé selon l'une des revendications 1 et 2, caractérisé en ce qu'on transforme un microorganisme, de préférence E. coli, avec un plasmide qui a été préparé selon la revendication 6.